**Europäisches Patentamt**

**European Patent Office**

**Office européen des brevets**

(19)

(11) Veröffentlichungsnummer : **0 373 103 B1**

(12) # EUROPÄISCHE PATENTSCHRIFT

(45) Veröffentlichungstag der Patentschrift :
**31.03.93 Patentblatt 93/13**

(51) Int. Cl.⁵ : **A61K 31/19, A61K 31/205**

(21) Anmeldenummer : **89810825.3**

(22) Anmeldetag : **01.11.89**

(54) **Flüssige orale Formulierung.**

Die Akte enthält technische Angaben, die nach dem Eingang der Anmeldung eingereicht wurden und die nicht in dieser Patentschrift enthalten sind.

(30) Priorität : **10.11.88 CH 4167/88**

(43) Veröffentlichungstag der Anmeldung :
**13.06.90 Patentblatt 90/24**

(45) Bekanntmachung des Hinweises auf die Patenterteilung :
**31.03.93 Patentblatt 93/13**

(84) Benannte Vertragsstaaten :
**AT BE CH DE ES FR GB GR IT LI LU NL SE**

(56) Entgegenhaltungen :
**FR-A- 2 315 948**
**GB-A- 2 134 529**
**CHEMICAL ABSTRACTS, Band 93, Nr. 24, 15. Dezember 1980, Seite 312, Zusammenfassung Nr. 225578q, Columbus, Ohio, US; C. LARSEN et al.: "Kinetics of the acid-catalyzed cyclization of diclofenac to an indolinone in aqueous solution"**

(73) Patentinhaber : **CIBA-GEIGY AG**
**Klybeckstrasse 141**
**CH-4002 Basel (CH)**

(72) Erfinder : **Affolter, Heidi, Dr.**
**Zwinglistrasse 29**
**CH-4127 Birsfelden (CH)**

## Beschreibung

Die Erfindung betrifft eine wässrige orale pharmazeutische Zubereitung von Diclofenac, ihre Verwendung und Herstellung.

Diclofenac mit der chemischen Bezeichnung o-(2,6-Dichloranilino)-phenylessigsäure ist als potentes Analgetikum und Antirheumatikum bekannt und wird beispielsweise im US Patent Nr. 3,558,690 beschrieben.

Ziel vielfacher Bemühungen war es, für diesen Wirkstoff eine wässrige, individuell dosierbare orale Darreichungsform zu entwickeln, die einen neutralen bzw. angenehmen Geschmack aufweist, und darüber hinaus in befriedigendem Masse lagerstabil ist, um dem Arzt sowie dem Patienten eine sinnvolle Alternative zu anderen oralen Zubereitungen zur Verfügung zu stellen.

Von Diclofenac ist bekannt, dass es einen bitteren Geschmack hat und ein kratzendes Gefühl im Hals hervorruft. Weiterhin hat sich als nachteilig herausgestellt, dass bei Verwendung üblicher wässriger oraler Lösungen schwer wasserlösliche Hydrate gebildet werden, die zu Ausfällungen führen. Ferner ist bekannt, dass Diclofenac insbesondere in saurer Lösung zum 1-(2,6-Dichlorphenyl)-indolin-2-on cyclisiert. Somit sind entsprechende wässrige pharmazeutische Formulierungen von Diclofenac nicht ausreichend lagerstabil und würden folglich nicht die Kriterien für die Registrierung als Arzneimittel erfüllen.

Der vorliegenden Erfindung lag die Aufgabe zugrunde, eine wässrige orale pharmazeutische Zubereitung von Diclofenac zu entwickeln, die die genannten Nachteile nicht aufweist.

Diese Aufgabe wird durch die erfindungsgemässe Zubereitung gelöst. Diese ist dadurch gekennzeichnet, dass Diclofenac in einem pH-Bereich von 2 bis 3,5 vorliegt.

Bei Verwendung derartiger wässriger Formulierungen wird überraschenderweise insbesondere keinerlei prohibitive Indolinonbildung festgestellt. Auch weisen solche Zubereitungen einen angenehmen Geschmack auf und erzeugen im Hals kein kratzendes Gefühl.

Die bevorzugte Wirkstoffkonzentration der erfindungsgemässen Zubereitung beträgt 0,5 bis 10 Gew.-%, insbesondere 1 bis 5 Gew.-%, in erster Linie etwa 1 Gew.-%.

Der erfindungsgemässe pH-Bereich liegt insbesondere zwischen 2 und 3,5, vorzugsweise zwischen 2,5 und 3,5, und wird durch an sich bekannte pharmazeutisch verwendbare Säuren bzw. Puffersysteme eingestellt. Als pharmazeutisch verwendbare Säuren kommen vorzugsweise Carbonsäuren, wie Essig-, Malon-, Fumar-, Malein-, Bernstein-, Milch-, Wein- oder in erster Linie Zitronensäure in Frage. Beispiele für derartige Puffersysteme sind Natriumcitrat/HCl- oder Zitronensäure/Phosphat-Puffer.

Entsprechende Applikationsformen der erfindungsgemässen Suspension sind Tropfen, Mixturen, Säfte oder Sirupe, die auch in Dosiseinheitsformen zur Anwendung gelangen können.

Die erfindungsgemässe Zusammensetzung kann weitere pharmazeutisch verwendbare Hilfs- und Zusatzstoffe enthalten, beispielsweise Konservierungsmittel, Antioxidantien, Aromen, Farbstoffe, Süssungsmittel, Suspensionsstabilisatoren und/oder Netzmittel.

Als Konservierungsmittel, die vor Befall von Mikroorganismen schützen, kommen Benzoesäure oder deren Salze, wie Natrium-, Kalium- oder Calciumsalze, 4-Hydroxy-benzoesäureester, wie 4-Hydroxybenzoesäuremethyl-, -ethyl- oder -propylester (PHB-Ester), Phenole, wie tert.-Butyl-4-methoxy- oder 2,6-Di-tert.-butyl-4-methyl-phenol, Phenylniederalkanole, Benzylalkohol, 4-Chlor- oder 2,4-Dichlorbenzylalkohol, 2-Phenylethanol, oder 3-Phenylpropanol, Chlorhexidindiacetat oder -digluconat, Thiabendazol, ferner Nitrofural, quartäre Ammoniumhalogenide, wie Alkoniumbromid, Benzalkoniumchlorid, Cetrimoniumbromid, Phenododeciniumbromid oder Cetylpyridiniumchlorid, oder in erster Linie Sorbinsäure, z.B. mit einem Anteil von 0,01 bis 0,1 Gew.-%, in Frage.

Geeignete Antioxidantien, die oxidative Prozesse inhibieren sollen, sind beispielsweise Sulfite, wie Alkalimetallsulfite, -hydrogensulfite oder -pyrosulfite, z.B. die entsprechenden Natrium- oder Kaliumsalze, Thiodipropionsäure, Thioglykol, Thiomilchsäure, Glutathion, vorzugsweise jedoch Cystein, Ascorbinsäure und deren Ester, z.B. Ascorbinsäuremyristat, -palmitat oder -stearat, oder Gallussäureester, wie -propyl-, -octyl- oder -dodecylester, z.B. mit einem Anteil von 0,01 bis 0,1 Gew.-%. Gegebenenfalls können Synergisten, wie Zitronen-, Citracon-, Phosphor- oder Weinsäure, beigemischt werden.

Zur Geschmacksverbesserung dienen als Aromen z.B. etherische Oele, insbesondere solche aus Früchten, wie Orangen, Pomeranzen-, Mandarinen-oder Citronenöle, ferner Zuckercouleur.

Geeignete Farbstoffe sind beispielsweise Indigotin I(blau), Amaranth(rot), Gelborange S (orange), Tartrazin XX (gelb) oder Chlorophyll (grün).

Als Süssungsmittel, welche z.B. in Sirupen in hoher Konzentration vorliegen, kommen beispielsweise Zucker, wie Mono- oder Disaccharide, z.B. D-Glucose, D-Fructose, D-Xylose, Maltose oder Saccharose, Polyole, wie Glycerin, Dulcitol, Mannitol, Sorbit oder Xylit, oder künstliche Süssstoffe, wie Saccharin oder das entsprechende Natrium-, Kalium- oder Calciumsalz, Cyclamat oder das entsprechende Natrium- oder Calciumsalz, Aspartam oder Acesulfam oder dessen Kaliumsalz, ferner Dulcin oder Ammoniumglycyrrhizinat.

Die Aufgabe der Stabilisatoren von Suspensionen besteht darin zu gewährleisten, dass die entnommenen Einzeldosen einen konstanten Wirkstoffgehalt aufweisen. Entsprechende Stabilisatoren sind z.B. anorganische Stabilisatoren von Suspensionen, z.B. kolloidale Silicate mit hohem Aluminium- und Magnesiumanteil, wie Bentonite, Veegum oder Gelwhite, kolloidale Kieselsäure, z.B. Aerosil® (Degussa), Cabosil® (Cabot), organische Stabilisatoren, z.B. Quellmittel, wie Alginate, z.B. Natriumalginat, Kaliumalginat oder Propylenglycolalginat, Gummi arabicum, Tragant, Karaya-Gummi, Sterculia-Gummi, Carageenan, Guar-Gummi oder Agar, synthetische oder halbsynthetische Quellmittel, z.B. 1,2-Epoxidpolymere, insbesondere Ethylenoxidhomopolymere mit einem Polymerisationsgrad von ca. 2 000-100 000, welche z.B. unter dem Handelsnamen Polyox® (Union Carbide) bekannt sind, vorzugsweise quellfähige Celluloseether, z.B. Methyl- oder Ethylcellulose, Hydroxyethylcellulose, Hydroxypropylcellulose, Hydroxypropylmethylcellulose, Methyl- oder Ethylhydroxyethylcellulose, Carboxymethylcellulose bzw. ein Alkalimetallsalz davon, oder mikrokristalline Cellulose, oder wasserlösliche Polyvinylverbindungen, wie Polyvinylacetat, Polyvinylalkohol oder Polyvinylpyrrolidon.

Vorteilhaft können den Stabilisatoren Netzmittel zugesetzt werden. Geeignete Netzmittel sind z.B. Sulfosuccinate wie Dihexyl-, Dioctyl- oder Diamylsulfosuccinat, Sulfonate oder Sulfate, z.B. Na-Alkylnaphthalinsulfonat, Fettalkoholsulfonate oder Fettalkoholpolyglycolethersulfate, Fettsäurepolyglycolester, z.B. Polyethylenglycolstearate, Polyglycolester von $C_8$-$C_{18}$-Fettsäuren, Fettalkoholpolyglycolether, z.B. Lauryl-, Cetyl-, Stearyl- oder Oleylalkoholpolyglycolether oder Cetylstearylalkoholpolyglycolether, Polyfettsäureesterpolyglycolether, z.B. Polyethylenglycolsorbitanmonolaurat, -monopalmitat, -monostearat oder -monooleat, Glycerinfettsäureesterpolyglycolether oder Pentaerythritfettsäurepolyglycolether, Saccharoseester, z.B. Saccharosemono- oder -distearat oder Saccharosemonopalmitat, ethoxylierte pflanzliche Oele, z.B. ethoxyliertes Rizinusöl oder hydriertes und ethoxyliertes Rizinusöl, oder Blockpolymerisate, wie Polyoxyethylen-Polyoxypropylen-Polymere.

Die erfindungsgemässen wässrigen oralen pharmazeutischen Zubereitungen von Diclofenac werden je nach Art der Applikationsform beispielsweise wie folgt hergestellt.

So wird beispielsweise das Wasser vorgelegt und gegebenenfalls unter Erwärmen, z.B. in einem Temperaturbereich von 20° bis 100°C, die Hilfs- und Zusatzstoffe eingearbeitet. In diese Grundlage wird dann der Wirkstoff suspendiert. Die Reihenfolge, in der die Bestandteile der erfindungsgemässen Zubereitung vermischt werden, ist jedoch unkritisch.

Zur Herstellung von Sirupen beispielsweise wird dem Wasser das Suspensionsmittel und der entsprechende Suspensionsstabilisator zugesetzt. Gegebenenfalls unter Erwärmen, z.B. in einem Temperaturbereich von 50° bis 100°C, werden Süssungsmittel sowie gewünschtenfalls Konservierungsmittel, Antioxidantien, Aromen und/oder Farbstoffe eingearbeitet. Anschliessend wird der Wirkstoff in das Medium suspendiert.

Die erfindungsgemässe wässrige orale pharmazeutische Zubereitung kann z.B. zur Behandlung von Schmerzzuständen und entzündlichen Prozessen verwendet werden.

Die Erfindung betrifft insbesondere auch die in den Beispielen beschriebenen Zubereitungen bzw. Herstellungsverfahren.

Die nachfolgenden Beispiele dienen lediglich der Erläuterung der vorstehend beschriebenen Erfindung; sie sollen diese jedoch in ihrem Umfang in keiner Weise einschränken.

Beispiel 1: Sirup, enthaltend 1 Gew.-% Diclofenac.

Zusammensetzung:

| | |
|---|---|
| Diclofenac (freie Säure) | 1,00 g |
| Hydroxyethylcellulose (Natrosol® 250 G) | 0,50 g |
| Cellulose und Natriumcarboxymethylcellulose (Avicel® RC 591) | 1,20 g |
| Sorbit-Lösung | 25,00 g |
| Sorbinsäure | 0,05 g |
| Vitamin C | 0,10 g |
| Zitronensäure | 0,20 g |

| | |
|---|---|
| Saccharin-Natrium | 0,06 g |
| Wasser entmineralisiert | 71,89 g |
| | 100,00 g |

pH-Wert:  2,9

Herstellung:

In das vorgelegte Wasser wird mit einem hochtourigen Mixer Avicel® suspendiert, Natrosol® dazugemischt und ca. 1 Std. ausquellen gelassen. Sorbit-Lösung und Sorbinsäure werden dazugegeben und unter Rühren auf ca. 85° erhitzt. Nach Abkühlen auf Raumtemperatur werden nacheinander Vitamin C, Zitronensäure und Saccharin unter Rühren darin gelöst. Mit dem Mixer wird der Wirkstoff darin suspendiert und das Gemisch anschliessend entlüftet.

Beispiel 2: Sirup enthaltend 1 % Diclofenac:

| | |
|---|---|
| Diclofenac (freie Säure) | 1,0 g |
| Saccharose | 40,0 g |
| Agar-Pulver | 0,3 g |
| Sorbinsäure | 0,1 g |
| Vitamin C | 0,1 g |
| Zitronensäure 1 AQ | 0,2 g |
| Dinatriumphosphat 2 AQ | 0,08 g |
| Zitronenaroma | 0,1 g |
| Wasser entmineralisiert | 58,12 g |
| | 100,00 g |

pH-Wert:  3,2

Herstellung:

Das Wasser wird auf ca. 90°C erwärmt und darin Sorbinsäure gelöst. Anschliessend wird Agarpulver eingestreut und mit einem hochtourigen Mixer dispergiert. Nun wird ca. 30 Minuten bei 90°C ausquellen gelassen. Die Saccharose wird zugegeben und unter Rühren gelöst. Nach Abkühlen auf ca. 40°C werden nacheinander Zitronensäure, Dinatriumphosphat und Vitamin C zugegeben und gelöst. Nach Zugabe des Aromas wird mit einem kleinen Teil der Grundlage der Wirkstoff angerieben und dann in dem Rest der Grundlage verteilt.

4

Beispiel 3: Mixtur enthaltend 1 % Diclofenac.

| | |
|---|---|
| Diclofenac (freie Säure) | 1,0 g |
| Hydroxyethylcellulose (Natrosol ® 250 G) | 0,5 g |
| Cellulose und Natriumcarboxymethylcellulose (Avicel ® RC 591) | 1,2 g |
| Sorbinsäure | 0,1 g |
| Vitamin C | 0,1 g |
| Zitronensäure 1 AQ | 0,2 g |
| Saccharin-Natrium | 0,06 g |
| Dinatriumphosphat 2 AQ | 0,08 g |
| Zitronenaroma | 0,1 g |
| Wasser entmineralisiert | 97,16 g |
| | 100,00 g |

pH-Wert: 3,2

Herstellung:

In 50 g Wasser wird mit einem hochtourigen Mixer Avicel® dispergiert, Natrosol® dazugemischt und ca. 1 Stunde ausquellen gelassen. Das restliche Wasser (47,16 g) wird auf ca. 60°C erhitzt und darin die Sorbinsäure gelöst. Nach Abkühlen auf 40°C werden nacheinander Saccharin-, Zitronensäure, Dinatriumphosphat und Vitamin C zugegeben und gelöst. Diese Lösung wird mit der Avicel®-Dispersion vermischt. Nach Zugabe des Aromas wird mit einem kleinen Teil der Grundlage der Wirkstoff angerieben und dann in dem Rest der Grundlage verteilt.

Beispiel 4: Tropfen enthaltend 1 % Diclofenac.

| | |
|---|---|
| Diclofenac (freie Säure) | 1,0 g |
| Meyprogat ® 150 (Guar Gummi) | 0,5 g |
| Sorbit-Lösung | 50,0 g |
| Sorbinsäure | 0,1 g |
| Vitamin C | 0,1 g |

| | |
|---|---|
| Zitronensäure 1 AQ | 0,2 g |
| Dinatriumphosphat 1 AQ | 0,08 g |
| Zitronenaroma | 0,1 g |
| Wasser entmineralisiert | 47,92 g |
| | 100,00 g |

pH-Wert: 3,1

Herstellung:

Das Wasser wird auf ca. 90°C erwärmt und darin Sorbinsäure gelöst. Meyprogat® wird eingestreut und mit einem hochtourigen Mixer dispergiert. Anschliessend wird ca. 30 Minuten bei 90°C ausquellen gelassen. Dann wird Sorbit-Lösung zugegeben. Nach Abkühlen auf ca. 40°C werden nacheinander Zitronensäure, Dinatriumphosphat und Vitamin C zugegeben und unter Rühren gelöst. Nach Zugabe des Aromas wird mit einem kleinen Teil der Grundlage der Wirkstoff angerieben und dann in dem Rest der Grundlage verteilt.

Beispiel 5: Sirup, enthaltend 1 Gew.-% Diclofenac.

| | |
|---|---|
| Diclofenac (freie Säure) | 1,00 g |
| Hydroxyethylcellulose (Natrosol® 250 G) | 0,50 g |
| Cellulose und Natriumcarboxymethylcellulose (Avicel® RC 591) | 1,20 g |
| Sorbit-Lösung | 25,00 g |
| Sorbinsäure | 0,05 g |
| Vitamin C | 0,60 g |
| Zitronensäure | 1,00 g |
| Saccharin-Natrium | 0,06 g |
| Wasser entmineralisiert | 70,59 g |
| | 100,00 g |

pH-Wert: 2,3

Herstellung:

In das vorgelegte Wasser wird mit einem hochtourigen Mixer Avicel® suspendiert, Natrosol® dazugemischt und ca. 1 Std. ausquellen gelassen. Sorbit-Lösung und Sorbinsäure werden dazugegeben und unter Rühren auf ca. 85° erhitzt. Nach Abkühlen auf Raumtemperatur werden nacheinander Vitamin C, Zitronensäure und Saccharin unter Rühren darin gelöst. Mit dem Mixer wird der Wirkstoff darin suspendiert und das Gemisch anschliessend entlüftet.

Beispiel 6: Sirup, enthaltend 1 Gew.-% Diclofenac.

| | |
|---|---|
| Diclofenac (freie Säure) | 1,00 g |
| Hydroxyethylcellulose (Natrosol® 250 G) | 0,50 g |
| Cellulose und Natriumcarboxymethylcellulose (Avicel® RC 591) | 1,20 g |
| Sorbit-Lösung | 25,00 g |
| Vitamin C | 0,60 g |
| Zitronensäure | 1,00 g |
| Methylparaben | 0,12 g |
| Propylparaben | 0,05 g |
| Saccharin-Natrium | 0,06 g |
| Wasser entmin. | 70,49 g |
| | 100,00 g |

pH-Wert: 3,0

Herstellung:

In das vorgelegte Wasser wird mit einem hochtourigen Mixer Avicel® suspendiert, Natrosol® dazugemischt und ca. 1 Std. ausquellen gelassen. Sorbit-Lösung und Sorbinsäure werden dazugegeben und unter Rühren auf ca. 85° erhitzt. Nach Abkühlen auf Raumtemperatur werden nacheinander Vitamin C, Zitronensäure und Saccharin unter Rühren darin gelöst. Mit dem Mixer wird der Wirkstoff darin suspendiert und das Gemisch anschliessend entlüftet.

**Patentansprüche**

**Patentansprüche für folgende Vertragsstaaten : AT, BE, CH, DE, FR, GB, IT, LI, LU, NL, SE**

1. Wässrige orale pharmazeutische Zubereitung von Diclofenac, dadurch gekennzeichnet, dass dass Diclofenac in suspendierter Form in eimem pH-Bereich von 2 bis 3,5 vorliegt.

2. Zubereitung nach Anspruch 1, dadurch gekennzeichnet, dass die Wirkstoffkonzentration 0,5 bis 10 Gew.-%, beträgt.

3. Zubereitung nach Anspruch 1 oder 2, dadurch gekennzeichnet, dass der pH-Bereich zwischen 2,5 und etwa 3,5 liegt.

4. Zubereitung nach Anspruch 3, dadurch gekennzeichnet, dass der pH-Wert durch Citronensäure eingestellt wird.

5. Zubereitung nach Anspruch 3, dadurch gekennzeichnet, dass der pH-Wert durch Citronensäure/Phosphatpuffer eingestellt wird.

6. Zubereitung nach einem der Ansprüche 1-5, dadurch gekennzeichnet, dass diese pharmazeutisch verwendbare Hilfs- und Zusatzstoffe ausgewählt aus der Gruppe der Konservierungsmittel, Antioxidantien, Aromen, Farbstoffe, Süssungsmittel, Suspensionsmittel und Netzmittel enthält.

7. Zubereitung nach Anspruch 6, dadurch gekennzeichnet, dass sie als Konservierungsmittel Benzoesäure oder deren Salze, 4-Hydroxybenzoesäureester, Phenole, Phenylniederalkanole, quartäre Ammoniumhalogenide oder Sorbinsäure enthält.

8. Zubereitung nach Anspruch 7, dadurch gekennzeichnet, dass sie Sorbinsäure enthält.

9. Zubereitung nach Anspruch 6, dadurch gekennzeichnet, dass sie als Antioxidans Cystein, Ascorbinsäure oder deren Ester oder einen Gallussäureester enthält.

10. Zubereitung nach Anspruch 9, dadurch gekennzeichnet, dass sie Ascorbinsäure enthält.

11. Zubereitung nach Anspruch 6, dadurch gekennzeichnet, dass sie als Süssungsmittel ein Mono- oder Disaccharid, Polyol oder einen künstlichen Süssstoff enthält.

12. Zubereitung nach Anspruch 6, dadurch gekennzeichnet, dass sie als Suspensionsstabilisator kolloidale Silicate mit hohem Aluminium- und Magnesiumanteil, kolloidale Kieselsäure, Quellmittel, quellfähige Celluloseether, Carboxymethylcellulose bzw. ein Alkalimetallsalz davon, mikrokristalline Cellulose oder wasserlösliche Polyvinylverbindungen enthält.

13. Zubereitung nach Anspruch 12, dadurch gekennzeichnet, dass sie Hydroxyethylcellulose und/oder Cellulose und Natriumcarboxymethylcellulose oder Agar oder Guar Gummi enthält.

14. Zubereitung nach einem der Ansprüche 1 - 13 in Form von Tropfen, Mixtur, Saft oder Sirup.

15. Zubereitung nach Anspruch 14 in Dosiseinheitsform.

16. Verfahren zur Herstellung einer wässrigen oralen pharmazeutischen Zubereitung von Diclofenac nach einem der Ansprüche 1 - 15, dadurch gekennzeichnet, dass man Diclofenac in Wasser suspendiert, den pH-Wert durch Säure bzw. Puffersystem einstellt und gegebenenfalls pharmazeutisch verwendbare Hilfs- und Zusatzstoffe beimischt.

17. Verfahren zur Herstellung einer wässrigen oralen pharmazeutischen Zubereintung von Diclofenac, dadurch gekennzeichnet, dass man Diclofenac in einem pH-Bereich von 2 bis 3,5 suspendiert und gegebenenfalls pharmazeutisch verwendbare Hilfs- und Zusatztoffe beimischt.

**Patentansprüche für folgende Vertragsstaaten : ES, GR**

1. Verfahren zur Herstellung einer wässrigen oralen pharmazeutischen Zubereintung von Diclofenac, dadurch gekennzeichnet, dass man Diclofenac in einem pH-Bereich von 2 bis 3,5 suspendiert und gegebenenfalls pharmazeutisch verwendbare Hilfs- und Zusatzstoffe beimischt.

2. Verfahren nach Anspruch 1, dadurch gekennzeichnet, dass man den Wirkstoff in einer Konzentration 0,5 bis 10 Gew.-%, einarbeitet.

3. Verfahren nach Anspruch 1 oder 2, dadurch gekennzeichnet, dass man einen pH-Bereich zwischen 2,5 und 3,5 einstellt.

4. Verfahren nach Anspruch 3, dadurch gekennzeichnet, dass der pH-Wert durch Citronensäure eingestellt wird.

5. Verfahren nach Anspruch 3, dadurch gekennzeichnet, dass der pH-Wert durch Citronensäure/Phosphatpuffer eingestellt wird.

6. Verfahren nach einem der Ansprüche 1-5, dadurch gekennzeichnet, dass man pharmazeutisch verwendbare Hilfs- und Zusatzstoffe ausgewählt aus der Gruppe der Konservierungsmittel, Antioxidantien, Aromen, Farbstoffe, Süssungsmittel, Suspensionsmittel und Netzmittel einarbeitet.

7. Verfahren nach Anspruch 6, dadurch gekennzeichnet, dass man als Konservierungsmittel Benzoesäure oder deren Salze, 4-Hydroxybenzoesäureester, Phenole, Phenylniederalkanole, quartäre Ammoniumhalogenide oder Sorbinsäure einarbeitet.

8. Verfahren nach Anspruch 7, dadurch gekennzeichnet, dass man Sorbinsäure einarbeitet.

**9.** Verfahren nach Anspruch 6, dadurch gekennzeichnet, dass man als Antioxidans Cystein, Ascorbinsäure oder deren Ester oder einen Gallussäureester einarbeitet.

**10.** Verfahren nach Anspruch 9, dadurch gekennzeichnet, dass man Ascorbinsäure einarbeitet.

**11.** Verfahren nach Anspruch 6, dadurch gekennzeichnet, dass man als Süssungsmittel ein Mono- oder Disaccharid, Polyol oder einen künstlichen Süssstoff einarbeitet.

**12.** Verfahren nach Anspruch 6, dadurch gekennzeichnet, dass man als Suspensionsstabilisator kolloidale Silicate mit hohem Aluminium- und Magnesiumanteil, kolloidale Kieselsäure, Quellmittel, quellfähige Celluloseether, Carboxymethylcellulose bzw. ein Alkalimetallsalz davon, mikrokristalline Cellulose oder wasserlösliche Polyvinylverbindungen einarbeitet.

**13.** Verfahren nach Anspruch 12, dadurch gekennzeichnet, dass man Hydroxyethylcellulose und/oder Cellulose und Natriumcarboxymethylcellulose oder Agar oder Guar Gummi einarbeitet.

**14.** Verfahren nach einem der Ansprüche 1 - 13, dadurch gekennzeichnet, dass man die Zubereitung in Form von Tropfen, Mixtur, Saft oder Sirup bereitstellt.

**15.** Verfahren nach Anspruch 14, dadurch gekennzeichnet, dass man die Zubereitung in Dosiseinheitsform bereitstellt.

## Claims

### Claims for the following Contracting States : AT, BE, CH, DE, FR, GB, IT, LI, LU, NL, SE

**1.** An aqueous oral pharmaceutical preparation of diclofenac, wherein diclofenac is present in suspended form in a pH range from 2 to 3.5.

**2.** A preparation according to claim 1, wherein the active ingredient concentration is from 0.5 to 10% by weight.

**3.** A preparation according to claim 1 or 2, wherein the pH range is between 2.5 and 3.5.

**4.** A preparation according to claim 3, wherein the pH is set using citric acid.

**5.** A preparation according to claim 3, wherein the pH is set using citric acid/phosphate buffer.

**6.** A preparation according to one of claims 1-5, which contains pharmaceutically utilizable adjuncts and additives selected from the group of preservatives, antioxidants, fragrances, dyes, sweeteners, suspension agents and wetting agents.

**7.** A preparation according to claim 6, which contains, as preservative, benzoic acid or salts thereof, 4-hydroxybenzoic acid esters, phenols, phenyl-lower alkanols, quaternary ammonium halides or sorbic acid.

**8.** A preparation according to claim 7, which contains sorbic acid.

**9.** A preparation according to claim 6, which contains, as antioxidant, cystein, ascorbic acid or esters thereof, or an ester of gallic acid.

**10.** A preparation according to claim 9, which contains ascorbic acid.

**11.** A preparation according to claim 6, which contains, as sweetener, a monosaccharide, disaccharide, polyol or artificial sweetener.

**12.** A preparation according to claim 6, which contains, as suspension stabilizer, colloidal silicates having a high aluminum and magnesium content, colloidal silica, swelling agents, swellable cellulose ethers, carboxymethylcellulose or an alkali metal salt thereof, microcrystalline cellulose or water-soluble polyvinyl compounds.

13. A preparation according to claim 12, which contains hydroxyethylcellulose and/or cellulose and sodium carboxymethylcellulose or agar or guar gum.

14. A preparation according to one of claims 1-13 in the form of drops, mixture, juice or syrup.

15. A preparation according to claim 14 in dose-unit form.

16. A method of preparing an aqueous oral pharmaceutical preparation of diclofenac according to one of claims 1-15, wherein diclofenac is suspended in water and the pH is set using acid and/or a buffer system and, optionally, pharmaceutically utilizable adjuncts and additives are admixed.

17. A method of preparing an aqueous oral pharmaceutical preparation of diclofenac, wherein diclofenac is suspended in a pH range from 2 to 3.5 and, optionally, pharmaceutically utilizable adjuncts and additives are admixed.

**Claims for the following Contracting States : ES, GR**

1. A method of preparing an aqueous oral pharmaceutical preparation of diclofenac, wherein diclofenac is suspended in a pH range from 2 to 3.5 and, optionally, pharmaceutically utilizable adjuncts and additives are admixed.

2. A method according to claim 1, wherein the active ingredient is incorporated in a concentration of from 0.5 to 10% by weight.

3. A method according to claim 1 or 2, wherein a pH range between 2.5 and 3.5 is set.

4. A method according to claim 3, wherein the pH is set using citric acid.

5. A method according to claim 3, wherein the pH is set using citric acid/phosphate buffer.

6. A method according to one of claims 1-5, which comprises incorporating pharmaceutically utilizable adjuncts and additives selected from the group of preservatives, antioxidants, fragrances, dyes, sweeteners, suspension agents and wetting agents.

7. A method according to claim 6, which comprises incorporating as preservative, benzoic acid or salts thereof, 4-hydroxybenzoic acid esters, phenols, phenyl-lower alkanols, quaternary ammonium halides or sorbic acid.

8. A method according to claim 7, which comprises incorporating sorbic acid.

9. A method according to claim 6, which comprises incorporating, as antioxidant, cystein, ascorbic acid or esters thereof, or an ester of gallic acid.

10. A method according to claim 9, which comprises incorporating ascorbic acid.

11. A method according to claim 6, which comprises incorporating, as sweetener, a monosaccharide, disaccharide, polyol or artificial sweetener.

12. A method according to claim 6, which comprises incorporating, as suspension stabilizer, colloidal silicates having a high aluminum and magnesium content, colloidal silica, swelling agents, swellable cellulose ethers, carboxymethylcellulose or an alkali metal salt thereof, microcrystalline cellulose or water-soluble polyvinyl compounds.

13. A method according to claim 12, which comprises incorporating hydroxyethylcellulose and/or cellulose and sodium carboxymethylcellulose or agar or guar gum.

14. A method according to one of claims 1-13, which comprises formulating the preparation in the form of drops, mixture, juice or syrup.

15. A method according to claim 14, which comprises formulating the preparation in dose-unit form.

## Revendications

**Revendications pour les Etats contractants suivants : AT, BE, CH, DE, FR, GB, IT, LI, LU, NL, SE**

1. Préparation pharmaceutique orale aqueuse de Diclofénac, caractérisée en ce que le Diclofénac se trouve sous une forme en suspension dans une plage de pH comprise entre 2 et 3,5.

2. Préparation selon la revendication 1, caractérisée en ce que la concentration en substance active est comprise entre 0,5 et 10 % en poids.

3. Préparation selon la revendication 1 ou 2, caractérisée en ce que la plage de pH est comprise entre 2,5 et 3,5.

4. Préparation selon la revendication 3, caractérisée en ce que le pH est ajusté par de l'acide citrique.

5. Préparation selon la revendication 3, caractérisée en ce que le pH est ajusté par le tampon acide citrique/phosphate.

6. Préparation selon l'une des revendications 1 à 5, caractérisée en ce qu'elle contient des adjuvants et additifs pharmaceutiquement utilisables choisis dans le groupe constitué par des conservateurs, antioxydants, arômes, colorants, édulcorants, agents de suspension et agents mouillants.

7. Préparation selon la revendication 6, caractérisée en ce qu'elle contient, comme conservateurs, l'acide benzoïque ou ses sels, les esters de l'acide 4-hydroxybenzoïque, les phénols, les phénylalcanols inférieurs, les halogénures d'ammonium quaternaire ou l'acide sorbique.

8. Préparation selon la revendication 7, caractérisée en ce qu'elle contient de l'acide sorbique.

9. Préparation selon la revendication 6, caractérisée en ce qu'elle contient, comme antioxydants, la cystéine, l'acide ascorbique ou ses esters ou un ester de l'acide gallique.

10. Préparation selon la revendication 9, caractérisée en ce qu'elle contient de l'acide ascorbique.

11. Préparation selon la revendication 6, caractérisée en ce qu'elle contient, comme édulcorant, un mono- ou disaccharide, un polyol ou un édulcorant artificiel.

12. Préparation selon la revendication 6, caractérisée en ce qu'elle contient, comme stabilisant de suspension, des silicates colloïdaux ayant une teneur élevée en aluminium et magnésium, de l'acide silicique colloïdal, des agents gonflants, des éthers cellulosiques aptes au gonflement, la carboxyméthylcellulose ou l'un de ses sels de métal alcalin, de la cellulose microcristalline ou des composés polyvinyliques solubles dans l'eau.

13. Préparation selon la revendication 12, caractérisée en ce qu'elle contient de l'hydroxyéthylcellulose et/ou de la cellulose et du carboxyméthylcellulose sodium ou de l'agar ou de la gomme de guar.

14. Préparation selon l'une des revendications 1 à 13 sous forme de gouttes, mélanges, jus ou sirop.

15. Préparation selon la revendication 14, sous forme de doses unitaires.

16. Procédé pour l'obtention d'une préparation pharmaceutique orale aqueuse de Diclofénac selon l'une des revendications 1 à 15, caractérisé en ce que l'on met le Diclofénac en suspension dans l'eau, en ce que l'on ajuste le pH par un acide ou un système de tampon et en ce que l'on ajoute éventuellement des adjuvants et additifs pharmaceutiquement utilisables.

17. Procédé pour l'obtention d'une préparation pharmaceutique orale aqueuse de Diclofénac, caractérisé en ce que l'on met en suspension le Diclofénac dans une plage de pH comprise entre 2 et 3,5 et en ce que l'on ajoute éventuellement des adjuvants et additifs pharmaceutiquement utilisables.

**Revendications pour les Etats contractants suivants : ES, GR**

1. Procédé pour l'obtention d'une préparation pharmaceutique orale aqueuse de Diclofénac, caractérisé en ce que l'on met en suspension le Diclofénac dans une plage de pH comprise entre 2 et 3,5 et en ce que l'on ajoute éventuellement des adjuvants et additifs pharmaceutiquement utilisables.

2. Procédé selon la revendication 1, caractérisé en ce que l'on ajoute la substance active en une concentration comprise entre 0,5 et 10 % en poids.

3. Procédé selon la revendication 1 ou 2, caractérisé en ce que l'on ajuste la plage de pH entre 2,5 et 3,5.

4. Procédé selon la revendication 3, caractérisé en ce que le pH est ajusté par l'acide citrique.

5. Procédé selon la revendication 3, caractérisé en ce que le pH est ajusté par le tampon acide citrique/phosphate.

6. Procédé selon l'une des revendications 1 à 5, caractérisé en ce que l'on ajoute des adjuvants et additifs pharmaceutiquement utilisables choisis dans le groupe constitué par des conservateurs, antioxydants, arômes, colorants, édulcorants, agents de suspension et agents mouillants.

7. Procédé selon la revendication 6, caractérisé en ce que l'on ajoute, comme conservateurs, l'acide benzoïque ou ses sels, les esters de l'acide 4-hydroxybenzoïque, les phénols, les phénylalcanols inférieurs, les halogénures d'ammonium quaternaire ou l'acide sorbique.

8. Procédé selon la revendication 7, caractérisé en ce que l'on ajoute de l'acide sorbique.

9. Procédé selon la revendication 6, caractérisé en ce que l'on ajoute, comme antioxydants, la cystéine, l'acide ascorbique ou ses esters ou un ester de l'acide gallique.

10. Procédé selon la revendication 9, caractérisé en ce que l'on ajoute de l'acide ascorbique.

11. Procédé selon la revendication 6, caractérisé en ce que l'on ajoute, comme édulcorant, un mono- ou disaccharide, un polyol ou un édulcorant artificiel.

12. Procédé selon la revendication 6, caractérisé en ce que l'on ajoute, comme stabilisant de suspension, des silicates colloïdaux ayant une teneur élevée en aluminium et magnésium, de l'acide silicique colloïdal, des agents gonflants, des éthers cellulosiques aptes au gonflement, la carboxyméthylcellulose ou l'un de ses sels de métal alcalin, de la cellulose microcristalline ou des composés polyvinyliques solubles dans l'eau.

13. Procédé selon la revendication 12, caractérisé en ce que l'on ajoute de l'hydroxyéthylcellulose et/ou de la cellulose et du carboxyméthylcellulose sodium ou de l'agar ou de la gomme de guar.

14. Procédé selon l'une des revendications 1 à 13, caractérisé en ce que la préparation est mise sous forme de gouttes, mélanges, jus ou sirop.

15. Procédé selon la revendication 14, caractérisé en ce que la préparation est mise sous forme de doses unitaires.